# EUROPEAN PATENT APPLICATION

(11) **EP 0 571 741 A2**
(43) Date of publication of application: **01.12.1993**
(21) Application number: 93105138.7
(22) Date of filing: 29.03.1993
(51) Int. Cl.: C12N 15/82, C12N 15/29, A01H 5/10

(54) **An expression plasmid for seeds**

(30) Priority: 27.03.1992 JP 70647/92
(71) Applicant: SUMITOMO CHEMICAL COMPANY, LIMITED, Osaka-shi, Osaka (JP)
(72) Inventor: Iida, Asako, Kobe-shi, Hyogo (JP); Nagasawa, Akitsu, Hyogo (JP); Oheda, Kenji, Kyoto-shi, Kyoto (JP)
(74) Representative: VOSSIUS & PARTNER

(57) **Abstract**

An expression plasmid constructed from a promoter and a terminator of a soybean seed storage protein glycinin gene.

The expression plasmid of the present invention serves to enhance the expression of plant genes in seeds and contributes to the development of industrial technology as regards plant breeding and production of valuable substances through the introduction and expression of useful plant genes.

## Description

The present invention relates to an expression plasmid for seeds. More particularly, the present invention is concerned with an expression plasmid constructed from a promoter and a GY terminator of a soybean seed storage protein glycinin gene (hereinafter abbreviated to "GY").

In various plant species, the development of a plasmid using a seed protein gene promoter has advanced for the purpose of expressing a foreign gene in seeds.

Glycinin is one of the main storage proteins occupying 20% or more of the whole seed protein of the soybean and is classified ,for the most part, into five types. Glycinin genes are designated as GY₁, GY₂, GY₃, GY₄ and GY₅ respectively, and properties thereof are described in The Plant Cell, Vol. 1, pp. 313-328 (1989).

At the present time, however, the expression ability of the foreign gene in seeds is not adequate, and expression plasmids, promoters and terminators for seeds, which enable the foreign gene to exhibit a higher expression ability, are desirable in the art.

Under the above-described circumstances, the present inventors have made an extensive study with the view to develop an excellent expression plasmid for seeds and, as a result, have found that an expression plasmid constructed from a promoter and a terminator obtained from certain storage protein genes in seeds has a higher expression ability than the conventional expression plasmid, which has led to the completion of the present invention.

Specifically, according to the present invention, there is provided an expression plasmid comprising a GY promoter and a GY terminator.

Further, according to a preferred embodiment of the present invention, there is provided an expression plasmid comprising the GY promoter and the GY terminator and a plurality of cloning sites carried in a region downstream of the promoter.

Further, according to another embodiment of the present invention, there is provided a plant cell or whole plant comprising said expression plasmid.

The expression plasmid of the present invention can serve to enhance the expression of plant genes in seeds and is useful for plant breeding and for the production of valuable substances through the introduction and expression of foreign genes.

### BRIEF DESCRIPTION OF THE DRAWINGS

Fig. 1 shows a restriction enzyme map of the expression plasmid pSUM-GY₁, wherein the oblique line portion represents a promoter of a soybean seed storage protein glycinin gene and the vertical line portion represents a terminator of a soybean seed storage protein glycinin gene;
Fig. 2 shows the base sequence of a multi-cloning site contained in plasmid pSUM-GY₁;
Fig. 3 shows step 1 for the construction of plasmid pSUM-GY₁;
Fig. 4 shows step 2 for the construction of plasmid pSUM-GY₁; and
Fig. 5 shows step 3 for the construction of plasmid pSUM-GY₁.

In general, an expression plasmid contains a promoter sequence that is bonded with a RNA polymerase, and initiates the transcription.

The expression plasmid of the present invention contains a GY promoter as a promoter. Examples of the GY promoter include a known gene comprising 690 base pairs described for instance in Nucleic Acids Research, Vol. 17, 4386 (1989) and is represented by the nucleotide sequence of SEQ ID No. 3 of the attached "SEQUENCE LISTING" and a novel gene comprising 1116 base pairs represented by the nucleotide sequence of SEQ ID No. 1.

The expression plasmid further comprises a terminator sequence at the 3' terminus to stop the transcription. In general, the promoter plays a more important role than the terminator as regards the expression of valuable genes. For this reason, in most plants, known terminators, for example, nopaline synthase (NOS) may be used, independent of whether the gene has the same origin as the promoter or belongs to the same origin as the promoter from the viewpoint of taxonomy (for example, plants, animals, bacteria, yeast, fungi and virus).

The expression plasmid of the present invention contains, as a terminator, a gene belonging to the same origin as the promoter, that is, a GY terminator.

Examples of the GY terminator include a novel gene comprising 744 base pairs represented by the nucleotide sequence of SEQ ID No. 2 of the attached "SEQUENCE LISTING". The terminator described herein is particularly useful for expression in the seeds of plants.

Within the meaning of the present invention the term "derived from a soybean seed storage protein glycinin gene" refers to promoter or terminator sequences of a soybean seed storage protein glycinin gene comprising nucleotide substitutions, deletions or additions with respect to the wild type sequences without altering the functional activity of the elements as determined in expression tests.

The expression plasmid of the present invention further carries, in a region downstream of the promoter, at least one, preferably two or more, cloning sites into which a given foreign gene can easily be inserted. The term "cloning site" used herein is intended to mean a site that can be recognized and cleaved with restriction enzymes used in conventional gene manipulations.

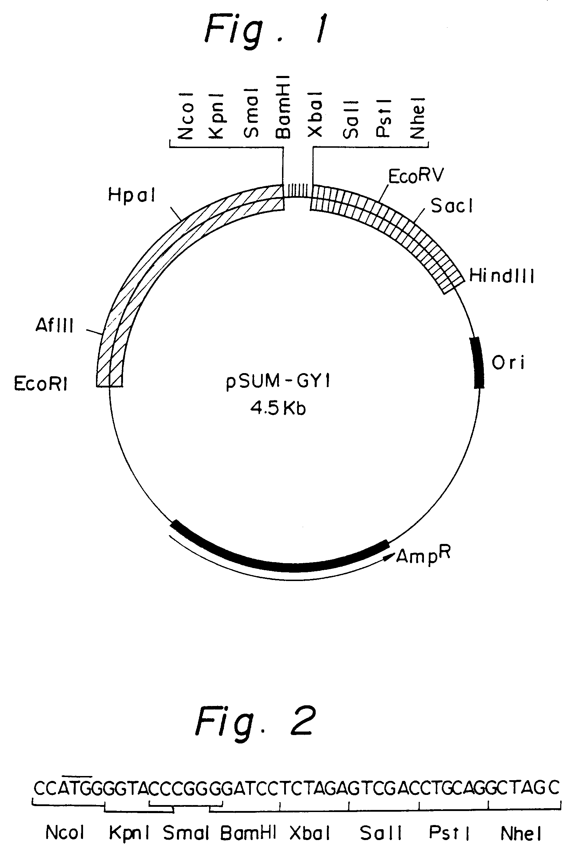

Specific examples of the cloning site, which can be carried in the expression plasmid of the present invention, include a gene represented by the nucleotide sequence of SEQ ID No. 4 in the attached "SEQUENCE LISTING". One example of the expression plasmid carrying such a cloning site is pSUM-GY₁ shown in Fig. 1.

Examples of useful foreign genes that can be introduced into plants with the aid of the expression plasmid according to the present invention, include (plant) storage protein genes, such as glycinin genes, β-conglycinin genes and other genes of soybean, which can improve the protein content of seeds when expressed in seeds, (plant) protein genes, such as 2S-albumin genes of Brazil nut, 10 kDa or 15 kDa protein genes of corn, 10 kDa protein genes of rice and 10 kDa protein genes of sunflower and other genes, which can improve the seeds so as to have a higher methionine content or a higher lysine content when expressed in seeds, biotin-biosynthesis-related enzyme genes, such as bioA, bioB, bioC, bioD, bioF, bioH, and a transcription regulatory factor, birA, of microorganisms including Escherichia coli, or genes encoding their homologous enzymes of plants, which can improve the seeds so as to have a higher biotin content when expressed in seeds, and related enzyme genes, such as stearoyl-ACP desaturase, acyl-ACP thioesterase and 3-phosphate acyl transferase, which can contribute to an increase in the oxidation stability of lipids and an improvement in seed lipids through a reduction in the phospholipid content or an increase in the oleic acid or linolenic acid content when expressed in seeds.

A selectable marker, for example, a drug-resistant gene, such as kanamycin or hygromycin, is added to the expression plasmid of the present invention capable of expressing the above-described valuable foreign genes, and the plasmid is then introduced into cells of dicotyledonous plants and monocotyledonous plants, such as soybean, tobacco , Arabidopsis, rice plant, corn, wheat, rapeseed and alfalfa, for example, by conventional direct gene transfer methods, such as a particle bombardment method, a polyethylene glycol (hereinafter referred to as "PEG") method wherein the plasmid DNA is incorporated into a protoplast by PEG, an electroporation method wherein the plasmid DNA is incorporated into a protoplast by applying an electric pulse and a microinjection method wherein the plasmid DNA is injected into plant cells with a micropipette, or a method wherein the expression plasmid of the present invention is once integrated within a T-DNA region of Ti plasmid or Ri plasmid of Agrobacterium and then introduced into a plant genome DNA.

The amount of expression plasmid of the present invention that is introduced into the plant cells is preferably about 1 to 10 ng per cell for the particle bombardment method, wherein use is made of a compressed-air-driver particle bombardment device (available from Rehbock Shoko Co., Ltd.), about 10 to 100 pg for the PEG method, about 10 to 100 pg per cell for the electroporation method, wherein an electric pulse of about 0.5 to 1 kV/cm is applied, and about 1 pg per cell for the microinjection method.

The plant cells, into which the expression plasmid of the present invention has been introduced, are cultured in a medium containing a drug as a selectable marker for the expression plasmid and a phytohormone, such as auxin, for about 1 to 2 months and further cultured in a regeneration medium for about 2 to 3 months to regenerate the whole plant.

In this case, it is possible to use known media used in the conventional plant culture, such as MS medium, White medium, B5 medium, N6 medium and NN medium.

As described above, the expression plasmid of the present invention can contribute to an improvement in the content of useful components in seeds, an improvement in the composition of the useful components and an improvement in the characteristic trait of plant seeds and enables useful substances to be produced in plant seeds by growing transformed plants wherein a foreign gene has been introduced and expressed.

The invention has been described in detail with particular reference to preferred embodiments thereof, but it will be understood that variations and modifications can be effected within the spirit and scope of the invention.

### Example 1: Construction of Plasmid pSUM-GY₁

Expression plasmid pSUM-GY₁ comprising a promoter region and a terminator region isolated from GY₁ and ligated respectively to both sides of a cloning site of plasmid pUC19 was constructed as follows.

At the outset, an about 700 bp gene fragment in a promoter region was amplified and isolated by a polymerase chain reaction (PCR) based on nucleotide sequence of GY₁ described in Nucleic Acids Research, Vol. 17, No. 11, p. 4386 (1989). A soybean genomic library (available from Clontech, Co. Ltd.) was subjected to screening by using this gene fragment as a probe, a gene fragment of about 4.4 kb considered to contain intact GY₁, that is, GY₁-#4, was subjected to subcloning, and the resultant plasmid was designated as "plasmid pGY₁-#4".

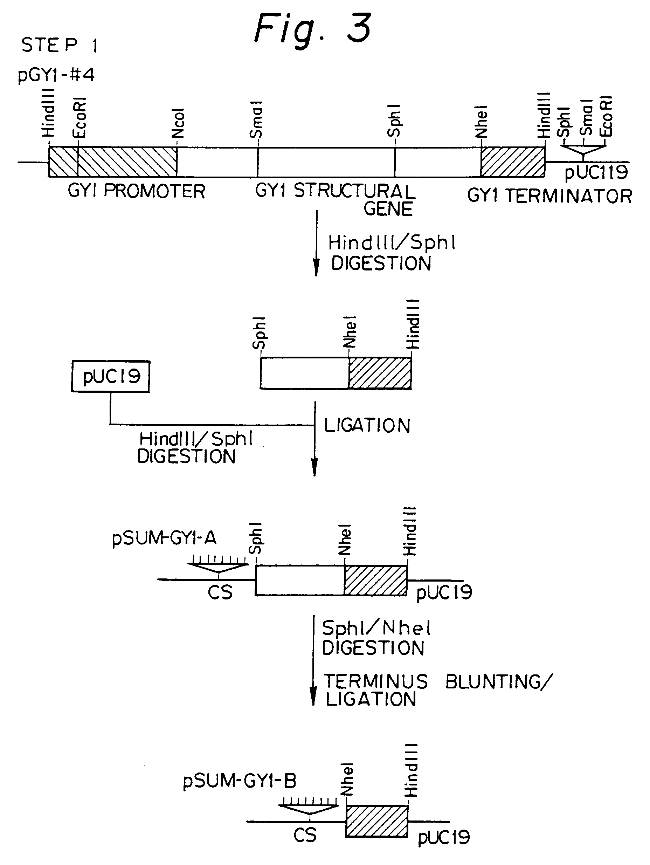

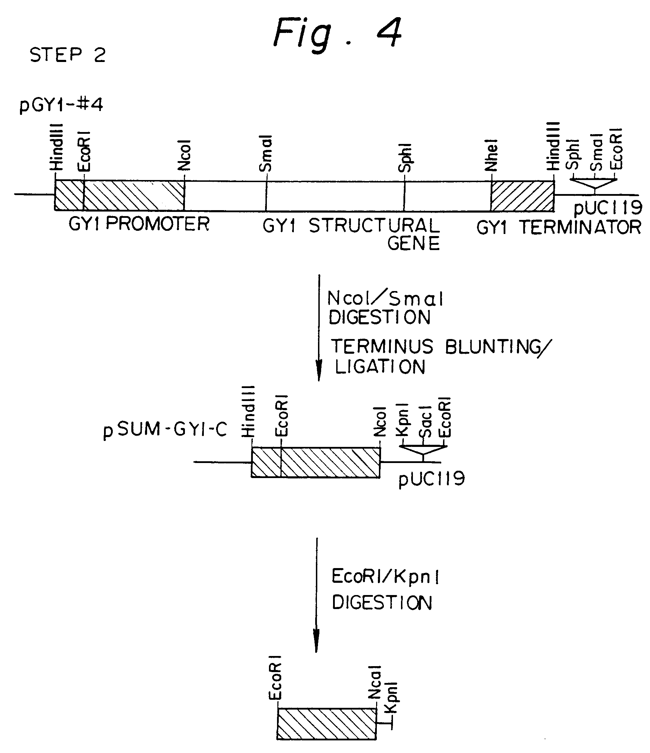

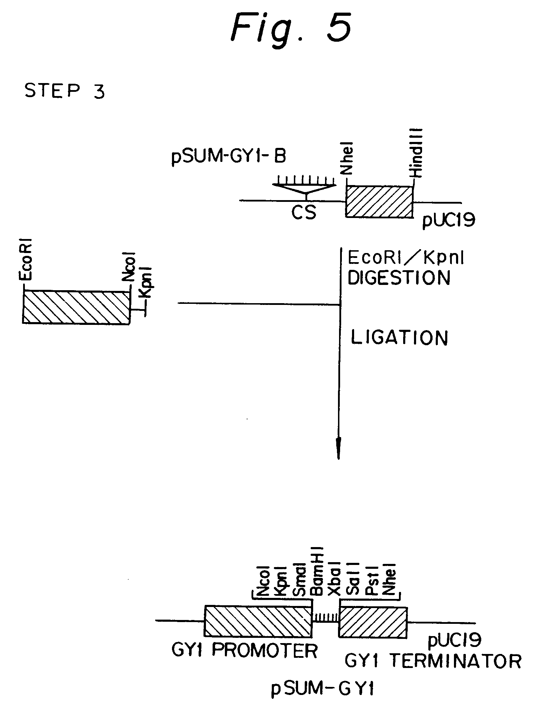

A promoter region and a terminator region were then isolated from plasmid pGY₁-#4 and ligated respectively to both sides of a cloning site of plasmid pUC19 so as to construct plasmid pSUM-GY₁. This is summarized in Figs. 3 to 5.

The construction method will now be described by the following three divided steps.

### Step 1: Construction of Plasmid pSUM-GY₁-B Containing the GY₁ Terminator

A reaction solution containing 1 µg of plasmid pGY₁-#4 and, added thereto, 10 units of restriction enzymes HindIII and SphI was allowed to react at 37°C for one hour, thereby digesting plasmid pGY₁-#4. The reaction mixture was then provided with 0.8% agarose gel containing 0.1 µg/ml ethidium bromide and subjected to electrophoresis. After the completion of electrophoresis, a gel portion corresponding to a 1.2 kb HindIII-SphI DNA fragment was cut out using ultraviolet irradiation, and the DNA fragment was purified by using a centrifugal tube equipped with a DNA recovery filter (available from Takara Shuzo Co., Ltd.). Separately, a reaction solution containing 1 µg of plasmid pUC19 and, added thereto, 10 units of restriction enzymes HindIII and SphI was allowed to react at 37°C for one hour. 0.2 µg of the HindIII-SphI DNA fragment of plasmid pUC19 and 0.2 µg of the 1.2 kb HindIII-SphI DNA fragment of plasmid pGY₁-#4 were mixed, and 5 units of T4 DNA ligase was added thereto so as to prepare a reaction solution that was then allowed to react at 16°C for 2 hr to effect ligation. Thereafter, according to a method established by Cohen et al. (see Proc. Natl. Acad. Sci. U.S.A. 69, (1972) pp. 2110-2114), the reaction mixture was transformed into E. coli HB101 strains (available from Takara Shuzo Co., Ltd.), and the transformant was spread over a LB plate containing 100 µg/ml ampicillin so as to isolate an ampicillin-resistant colony. A plasmid was then prepared from the colony according to a method established by Birnboim et al. (see Nucl. Acids. Res., 7, (1979) pp. 1513-1523), and a reaction solution containing 1 µg of the plasmid and, added thereto, 5 units of restriction enzyme NheI was allowed to react at 37°C for one hour. The reaction mixture was subjected to agarose gel electrophoresis so as to select a plasmid cleaved at its one site with restriction enzyme NheI. This plasmid was designated as "plasmid pSUM-GY₁-A". A reaction solution containing 1 µg of plasmid pSUM-GY₁-A and, added thereto, 10 units of restriction enzymes SphI and NheI was allowed to react at 37°C for one hour. DNA was purified by precipitation with ethanol, and about 0.1 µg of the purified DNA at its end was blunted with T4 DNA polymerase and then subjected to ligation with T4 ligase by using DNA Blunting Kit available from Takara Shuzo Co., Ltd. Thereafter, according to the method established by Cohen et al., the reaction mixture was transformed into E. coli HB101 strains, and an ampicillin-resistant colony was isolated. A plasmid was then prepared from the colony according to the method established by Birnboim et al. A reaction solution containing 1 µg of the plasmid and, added thereto, 5 units of restriction enzymes SphI or NheI was allowed to react at 37°C for one hour. The reaction mixture was subjected to agarose gel electrophoresis so as to select a plasmid cleaved at its one site with restriction enzyme NheI but not cleaved with restriction enzyme SphI. This plasmid was designated as "plasmid pSUM-GY₁-B". The plasmid pSUM-GY₁-B had a cloning site derived from plasmid pUC19 and a GY₁ terminator of about 0.7 kb.

### Step 2: Preparation of a DNA fragment containing the GY₁ Promoter

A reaction solution containing 1 µg of plasmid pGY₁-#4 and, added thereto, 10 units of restriction enzymes NcoI and SmaI was allowed to react at 37°C for one hour. The DNA was purified by precipitation with ethanol, and about 0.1 µg of the purified DNA at its end was blunted with T4 DNA polymerase and subjected to ligation with T4 ligase. According to the method established by Cohen et al., the reaction mixture was transformed into E.coli HB101 strains, and an ampicillin-resistant colony was isolated. A plasmid was then prepared from the colony according to the method established by Birnboim et al. A reaction solution containing 1 µg of the plasmid and, added thereto, 5 units of restriction enzymes NcoI or SmaI was allowed to react at 37°C for one hour. The reaction mixture was subjected to agarose gel electrophoresis so as to select a plasmid cleaved at its one site with restriction enzyme NcoI but not cleaved with restriction enzyme SmaI. This plasmid was designated as "plasmid pSUM-GY₁-C". Further, a reaction solution containing 1 µg of plasmid pGpSUM-GY₁-C and, added thereto, 10 units of restriction enzymes EcoRI and KpnI was allowed to react at 37°C for one hour. The reaction mixture was subjected to electrophoresis, and a gel portion corresponding to a 1.1 kb EcoRI-KpnI DNA was cut out, and the DNA fragment was purified by using a centrifugal tube equipped with a DNA recovery filter (available from Takara Shuzo Co., Ltd.).

### Step 3: Construction of Plasmid pSUM-GY₁

A reaction solution containing plasmid pSUM-GY₁-B prepared in Step 1 and, added thereto, 10 units of restriction enzymes EcoRI and KpnI was allowed to react at 37°C for one hour. 0.2 µg of the EcoRI-KpnI DNA fragment of plasmid pSUM-GY₁-B and 0.2 µg of the approximately 1.1 kb EcoRI-KpnI DNA fragment containing the GY₁ promoter were mixed, and 5 units of T4 DNA ligase was added thereto to prepare a reaction solution that was then allowed to react at 16°C for 2 hr so as to effect ligation. Thereafter, according to the method established by Cohen et al., the reaction mixture was transformed into E. coli HB101 strains, and an ampicillin-resistant colony was isolated. A plasmid was then prepared from the colony according to the method established by Birnboim et al. A reaction mixture containing 1 µg of the plasmid and, added thereto, 5 units of restriction enzymes EcoRI and HindIII was allowed to react at 37°C for one hour. The reaction mixture was subjected to agarose gel electrophoresis to select a plasmid from which two DNA fragments, that is, an approximately 1.8 kb DNA fragment and an approximately 2.7 kb DNA fragment, were detected. This plasmid was designated as plasmid "pSUM-GY₁". The constructed expression plasmid pSUM-GY₁ was forwarded as Escherichia coli HB101/pSUM-GY₁ on March 26, 1992 (Registered Mail Receipt No. 161 38 785294) to the Patent Microorganism Depository, Fermentation Research Institute, Agency of Industrial Science and Technology, and deposited as FERM BP-4131 under Budapest Treaty with said Patent Microorganism Depository (address: 1-3, Higashi 1 chome, Tsukuba-shi, Ibaraki-ken, 305, Japan) as the International Depository Authority.

### Example 2: Determination of Promoter/Terminator Activity of the Constructed Expression Plasmid PSUM-GY₁

A GUS gene as a reporter gene was inserted into a cloning site of the constructed expression plasmid pSUM-GY₁, and the expression of the gene was examined in an immature seed of a soybean so as to determine the GY₁ promoter/terminator activity. The method will now be described in detail.

A 1.87 kb GUS gene fragment derived from plasmid pRAJ225 (available from Clontech Co. Ltd.) was inserted into between NcoI and NheI within the multi-cloning site of plasmid pSUM-GY₁ so as to construct the GUS expression plasmid pSUM-GY-001.

Plasmid pSUM-GY₁-001 was introduced into an immature seed of a soybean 1 to 2 months after flowering together within an internal standard plasmid pD0432 (see Science, 234, 856-859 (1986)) comprising luciferase (LUC) gene of a firefly ligated downstream of a cauliflower mosaic virus (CaMV) 35S promoter by a particle bombardment method (see Shokubutsu Saibo Kogaku, 2, pp. 631-637 (1990)). Separately, GUS expression plasmid pGY1100 having the same structure as plasmid-pSUM-GY₁-001, except for the replacement of the GY₁ terminator with a nopaline synthase gene (NOS) terminator, was introduced in the same manner as that described above. 24 to 48 hrs after the introduction of the plasmid, the GUS/LUC activity was determined according to a method established by Morikawa et al. (see Appl. Microbiol. Biotechnol., 31, pp. 320-322 (1989)). The results are given in Table 1.

**Table 1**

| Plasmid | Promoter | Terminator | LUC Activity (light units/µg protein) | GUS Activity (pmole/h/µg protein) | Specific Activity (GUS/LUC × 10⁴) | |
|---|---|---|---|---|---|---|
| | | | | | | Average |
| pSUMGY₁ -001 | GY | GY | 8.6 | 0.019 | 22.2 | 27.8±7.5 |
| | | | 39.6 | 0.084 | 21.2 | |
| | | | 42.5 | 0.119 | 27.9 | |
| | | | 7.5 | 0.030 | 40.0 | |
| pGY1100 | GY | NOS | 72.0 | 0.06 | 8.9 | 9.1±1.9 |
| | | | 105.5 | 0.064 | 6.1 | |
| | | | 31.0 | 0.031 | 10.1 | |
| | | | 65.6 | 0.074 | 11.3 | |

When plasmid pSUM-GY₁-001 was introduced, the GUS/LUC activity was about three times that for the introduction of plasmid pGY1100. This demonstrates that the GY₁ promoter/terminator has an activity in the tissue of an immature seed and, in the seed tissue, the novel GY₁ terminator provides a higher expression ability than a known NOS terminator widely used in conventional plants.

SEQUENCE LISTING
INFORMATION FOR SEQ ID NO: 1
(i) SEQUENCE CHARACTERISTICS:
(A) LENGTH: 1116 base pairs
(B) TYPE: nucleic acid
(C) STRANDEDNESS: double-stranded
(D) TOPOLOGY: linear
(E) FEATURES
Definition: promoter
Location: 1 ... 1116
Identification Method: E
(ii) SEQUENCE DESCRIPTION: FOR SEQ ID NO: 1:
GAATTCTCTC TTATAAAACA CAAACACAAT TTTTAGATTT TATTTAAATA ATCATCAATC 60
GATTATAATT ATTTATATAT TTTTCTATTT TCAAAGAAGT AAATCATGAG CTTTTCCAAC 120
TCAACATCTA TTTTTTTTCT CTCAACCTTT TTCACATCTT AAGTAGTCTC ACCCTTTATA 180
TATATAACTT ATTTCTTACC TTTTACATTA TGTAACTTTT ATCACCAAAA CCAACAACTT 240
TAAAATTTTA TTAAATAGAC TCCACAAGTA ACTTGACACT CTTACATTCA TCGACATTAA 300
CTTTTATCTG TTTTATAAAT ATTATTGTGA TATAATTTAA TCAAAATAAC CACAAACTTT 360
CATAAAAGGT TCTTATTAAG CATGGCATTT AATAAGCAAA AACAACTCAA TCACTTTCAT 420
ATAGGAGGTA GCCTAAGTAC GTACTCAAAA TGCCAACAAA TAAAAAAAAA GTTGCTTTAA 480
TAATGCCAAA ACAAATTAAT AAAACACTTA CAACACCGGA TTTTTTTTAA TTAAAATGTG 540
CCATTTAGGA TAAATAGTTA ATATTTTTAA TAATTATTTA AAAAGCCGTA TCTACTAAAA 600
TGATTTTTAT TTGGTTGAAA ATATTAATAT GTTTAAATCA ACACAATCTA TCAAAATTAA 660
ACTAAAAAAA AAATAAGTGT ACGTGGTTAA CATTAGTACA GTAATATAAG AGGAAAATGA 720
GAAATTAAGA AATTGAAAGC GAGTCTAATT TTTAAATTAT GAACCTGCAT ATATAAAAGG 780
AAAGAAAGAA TCCAGGAAGA AAAGAAATGA AACCATGCAT GGTCCCCTCG TCATCACGAG 840
TTTCTGCCAT TTGCAATAGA AACACTGAAA CACCTTTCTC TTTGTCACTT AATTGAGATG 900
CCGAAGCCAC CTCACACCAT GAACTTCATG AGGTGTAGCA CCCAAGGCTT CCATAGCCAT 960
GCATACTGAA GAATGTCTCA AGCTCAGCAC CCTACTTCTG TGACGTGTCC CTCATTCACC 1020
TTCCTCTCTT CCCTATAAAT AACCACGCCT CAGGTTCTCC GCTTCACAAC TCAAACATTC 1080
TCTCCATTGG TCCTTAAACA CTCATCAGTC ATCACC 1116

INFORMATION FOR SEQ ID NO: 2
(i) SEQUENCE CHARACTERISTICS:
(A) LENGTH: 744 base pairs
(B) TYPE: nucleic acid
(C) STRANDEDNESS: double-stranded

(D) TOPOLOGY: linear
(E) FEATURES
Definition: terminator
Location: 1 ... 744
Identification Method: E
(ii) SEQUENCE DESCRIPTION: FOR SEQ ID NO: 2:
CTAGCAACCA ATAAATAATA ATAATAATAA TGAATAAGAA AACAAAGGCT TTAGCTTGCC 60
TTTTGTTCAC TGTAAAATAA TAATGTAAGT ACTCTCTATA ATGAGTCACG AAACTTTTGC 120
GGGAATAAAA GGAGAAATTC CAATGAGTTT TCTGTCAAAT CTTCTTTTGT CTCTCTCTCT 180
CTCTCTTTTT TTTTTCTTTC TTCTGAGCTT CTTGCAAAAC AAAAGGCAAA CAATAACGAT 240
TGGTCCAATG ATAGTTAGCT TGATCGATGA TATCTTTAGG AAGTGTTGGC AGGACAGGAC 300
ATGATGTAGA AGACTAAAAT TGAAAGTATT GCAGACCCAA TAGTTGAAGA TTAACTTTAA 360
GAATGAAGAC GTCTTATCAG GTTCTTCATG ACTTGGAGCT CAACCCAACT TGGAAAGTTC 420
GAGAGTATTT GGACCATTGT GCTTTGTGTC TTCAAACATA AAACATCGCT CCAAATTTAA 480
CATGGGAGCT AAAAAATGTG TTTTTCTGGG ATTTTAATTT TCAACAGAGT CAAGGATGGT 540
GTTGCATATG ATGTCTTGAT GTCCATTGTC CACACTAAAT AGATATTGGT TTCAAGAAAT 600
ATTAATTTCA TTTTCATGAC TTTCAATTCA TAAACCTTAA ACGAATATTA ATTTAAAATC 660
TATCCTCAAA TGATAAATTT TAAAAAAAAT TACCCCCAAT CGGTAATTTG ACTCACAAGT 720
TAGTTAGTTG ATATTTTGAA GCTT 744

INFORMATION FOR SEQ ID NO: 3
(i) SEQUENCE CHARACTERISTICS:
(A) LENGTH: 690 base pairs
(B) TYPE: nucleic acid
(C) STRANDEDNESS: double-stranded
(D) TOPOLOGY: linear
(E) FEATURES
Definition: promoter
Location: 1 ... 690
Identification Method: E
(ii) SEQUENCE DESCRIPTION: FOR SEQ ID NO: 3:
TAGCCTAAGT ACGTACTCAA AATGCCAACA AATAAAAAAA AAGTTGCTTT AATAATGCCA 60
AAACAAATTA ATAAAACACT TACAACACCG GATTTTTTTT AATTAAAATG TGCCATTTAG 120
GATAAATAGT TAATATTTTT AATAATTATT TAAAAAGCCG TATCTACTAA AATGATTTTT 180
ATTTGGTTGA AAATATTAAT ATGTTTAAAT CAACACAATC TATCAAAATT AAACTAAAAA 240
AAAAATAAGT GTACGTGGTT AACATTAGTA CAGTAATATA AGAGGAAAAT GAGAAATTAA 300
GAAATTGAAA GCGAGTCTAA TTTTTAAATT ATGAACCTGC ATATATAAAA GGAAAGAAAG 360

AATCCAGGAA GAAAAGAAAT GAAACCATGC ATGGTCCCCT CGTCATCACG AGTTTCTGCC 420
ATTTGCAATA GAAACACTGA AACACCTTTC TCTTTGTCAC TTAATTGAGA TGCCGAAGCC 480
ACCTCACACC ATGAACTTCA TGAGGTGTAG CACCCAAGGC TTCCATAGCC ATGCATACTG 540
AAGAATGTCT CAAGCTCAGC ACCCTACTTC TGTGACGTTG TCCCTCATTC ACCTTCCTCT 600
CTTCCCTATA AATAACCACG CCTCAGGTTC TCCGCTTCAC AACTCAAACA TTCTCCTCCA 660
TTGGTCCTTA AACACTCATC AGTCATCACC 690

INFORMATION FOR SEQ ID NO: 4
(i) SEQUENCE CHARACTERISTICS:
(A) LENGTH: 45 base pairs
(B) TYPE: nucleic acid
(C) STRANDEDNESS: double-stranded
(D) TOPOLOGY: linear
(ii) SEQUENCE DESCRIPTION: FOR SEQ ID NO: 4:
CCATGGGGTA CCCGGGGATC CTCTAGAGTC GACCTGCAGG CTAGC 45

SEQUENCE LISTING

(1) GENERAL INFORMATION:

(i) APPLICANT:
(A) NAME: Sumitomo Chemical Company, Ltd.
(B) STREET: 5-33, Kitahama 4-chome, Chuo-ku
(C) CITY: Osaka-shi, Osaka
(E) COUNTRY: Japan
(F) POSTAL CODE (ZIP): none

(ii) TITLE OF INVENTION: An Expression Plasmid for Seeds

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(v) CURRENT APPLICATION DATA:
APPLICATION NUMBER: EP 93 10 5138.7

(2) INFORMATION FOR SEQ ID NO: 1:

(i) SEQUENCE CHARACTERISTICS:
(A) LENGTH: 1116 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iii) ANTI-SENSE: NO

(ix) FEATURE:
(A) NAME/KEY: promoter
(B) LOCATION: 1..1116

(xi) SEQUENCE DESCRIPTION: SEQ ID NO: 1:

GAATTCTCTC TTATAAAACA CAAACACAAT TTTTAGATTT TATTTAAATA ATCATCAATC 60

GATTATAATT ATTTATATAT TTTTCTATTT TCAAAGAAGT AAATCATGAG CTTTTCCAAC 120

TCAACATCTA TTTTTTTTCT CTCAACCTTT TTCACATCTT AAGTAGTCTC ACCCTTTATA 180

TATATAACTT ATTTCTTACC TTTTACATTA TGTAACTTTT ATCACCAAAA CCAACAACTT 240

TAAAATTTTA TTAAATAGAC TCCACAAGTA ACTTGACACT CTTACATTCA TCGACATTAA 300

CTTTTATCTG TTTTATAAAT ATTATTGTGA TATAATTTAA TCAAAATAAC CACAAACTTT 360

CATAAAAGGT TCTTATTAAG CATGGCATTT AATAAGCAAA AACAACTCAA TCACTTTCAT 420

ATAGGAGGTA GCCTAAGTAC GTACTCAAAA TGCCAACAAA TAAAAAAAAA GTTGCTTTAA 480

TAATGCCAAA ACAAATTAAT AAAACACTTA CAACACCGGA TTTTTTTTAA TTAAAATGTG 540

CCATTTAGGA TAAATAGTTA ATATTTTTAA TAATTATTTA AAAAGCCGTA TCTACTAAAA 600

TGATTTTTAT TTGGTTGAAA ATATTAATAT GTTTAAATCA ACACAATCTA TCAAAATTAA 660

ACTAAAAAAA AAATAAGTGT ACGTGGTTAA CATTAGTACA GTAATATAAG AGGAAAATGA 720

GAAATTAAGA AATTGAAAGC GAGTCTAATT TTTAAATTAT GAACCTGCAT ATATAAAAGG 780

AAAGAAAGAA TCCAGGAAGA AAAGAAATGA AACCATGCAT GGTCCCCTCG TCATCACGAG 840

TTTCTGCCAT TTGCAATAGA AACACTGAAA CACCTTTCTC TTTGTCACTT AATTGAGATG 900

CCGAAGCCAC CTCACACCAT GAACTTCATG AGGTGTAGCA CCCAAGGCTT CCATAGCCAT 960

GCATACTGAA GAATGTCTCA AGCTCAGCAC CCTACTTCTG TGACGTGTCC CTCATTCACC 1020

TTCCTCTCTT CCCTATAAAT AACCACGCCT CAGGTTCTCC GCTTCACAAC TCAAACATTC 1080

TCTCCATTGG TCCTTAAACA CTCATCAGTC ATCACC 1116

(2) INFORMATION FOR SEQ ID NO: 2:

(i) SEQUENCE CHARACTERISTICS:
(A) LENGTH: 744 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iii) ANTI-SENSE: NO

(ix) FEATURE:
(A) NAME/KEY: terminator
(B) LOCATION: 1..744

(xi) SEQUENCE DESCRIPTION: SEQ ID NO: 2:

CTAGCAACCA ATAAATAATA ATAATAATAA TGAATAAGAA AACAAAGGCT TTAGCTTGCC 60

TTTTGTTCAC TGTAAAATAA TAATGTAAGT ACTCTCTATA ATGAGTCACG AAACTTTTGC 120

GGGAATAAAA GGAGAAATTC CAATGAGTTT TCTGTCAAAT CTTCTTTTGT CTCTCTCTCT 180

CTCTCTTTTT TTTTTCTTTC TTCTGAGCTT CTTGCAAAAC AAAAGGCAAA CAATAACGAT 240

TGGTCCAATG ATAGTTAGCT TGATCGATGA TATCTTTAGG AAGTGTTGGC AGGACAGGAC 300

ATGATGTAGA AGACTAAAAT TGAAAGTATT GCAGACCCAA TAGTTGAAGA TTAACTTTAA 360

GAATGAAGAC GTCTTATCAG GTTCTTCATG ACTTGGAGCT CAACCCAACT TGGAAAGTTC 420

GAGAGTATTT GGACCATTGT GCTTTGTGTC TTCAAACATA AAACATCGCT CCAAATTTAA 480

CATGGGAGCT AAAAAATGTG TTTTTCTGGG ATTTTAATTT TCAACAGAGT CAAGGATGGT 540

GTTGCATATG ATGTCTTGAT GTCCATTGTC CACACTAAAT AGATATTGGT TTCAAGAAAT 600

ATTAATTTCA TTTTCATGAC TTTCAATTCA TAAACCTTAA ACGAATATTA ATTTAAAATC 660

TATCCTCAAA TGATAAATTT TAAAAAAAAT TACCCCCAAT CGGTAATTTG ACTCACAAGT 720

TAGTTAGTTG ATATTTTGAA GCTT 744

(2) INFORMATION FOR SEQ ID NO: 3:

(i) SEQUENCE CHARACTERISTICS:
(A) LENGTH: 690 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iii) ANTI-SENSE: NO

(ix) FEATURE:
(A) NAME/KEY: promoter
(B) LOCATION: 1..690

(xi) SEQUENCE DESCRIPTION: SEQ ID NO: 3:

TAGCCTAAGT ACGTACTCAA AATGCCAACA AATAAAAAAA AAGTTGCTTT AATAATGCCA 60

AAACAAATTA ATAAAACACT TACAACACCG GATTTTTTTT AATTAAAATG TGCCATTTAG 120

GATAAATAGT TAATATTTTT AATAATTATT TAAAAAGCCG TATCTACTAA AATGATTTTT 180

ATTTGGTTGA AAATATTAAT ATGTTTAAAT CAACACAATC TATCAAAATT AAACTAAAAA 240

AAAAATAAGT GTACGTGGTT AACATTAGTA CAGTAATATA AGAGGAAAAT GAGAAATTAA 300

GAAATTGAAA GCGAGTCTAA TTTTTAAATT ATGAACCTGC ATATATAAAA GGAAAGAAAG 360

AATCCAGGAA GAAAAGAAAT GAAACCATGC ATGGTCCCCT CGTCATCACG AGTTTCTGCC 420

ATTTGCAATA GAAACACTGA AACACCTTTC TCTTTGTCAC TTAATTGAGA TGCCGAAGCC 480

ACCTCACACC ATGAACTTCA TGAGGTGTAG CACCCAAGGC TTCCATAGCC ATGCATACTG 540

AAGAATGTCT CAAGCTCAGC ACCCTACTTC TGTGACGTTG TCCCTCATTC ACCTTCCTCT 600

CTTCCCTATA AATAACCACG CCTCAGGTTC TCCGCTTCAC AACTCAAACA TTCTCCTCCA 660

TTGGTCCTTA AACACTCATC AGTCATCACC 690

(2) INFORMATION FOR SEQ ID NO: 4:

(i) SEQUENCE CHARACTERISTICS:
(A) LENGTH: 45 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iii) ANTI-SENSE: NO

(xi) SEQUENCE DESCRIPTION: SEQ ID NO: 4:

CCATGGGGTA CCCGGGGATC CTCTAGAGTC GACCTGCAGG CTAGC 45

## Claims

1. An expression plasmid comprising a promoter derived from a soybean seed storage protein glycinin gene and a terminator derived from a soybean seed storage protein glycinin gene.

2. The expression plasmid according to claim 1, wherein said promoter is represented by the nucleotide sequence of SEQ ID No. 1 and said terminator is represented by the nucleotide sequence of SEQ ID No. 2.

3. The expression plasmid according to any one of claims 1 or 2, wherein at least one cloning site is carried in a region downstream of the promoter.

4. The expression plasmid according to claim 3, wherein said cloning site contains the nucleotide sequence of SEQ ID No. 4.

5. The expression plasmid according to claim 3, which is specified by plasmid pSUM-GY₁ (FERM BP-4131).

6. A promoter of a soybean seed storage protein glycinin gene represented by the nucleotide sequence of SEQ ID No. 1.

7. A terminator of a soybean seed storage protein glycinin gene represented by the nucleotide sequence of SEQ ID No. 2.

8. A method for expression of a foreign gene in a seed, plant cell or plant comprising introducing a useful foreign gene into an expression plasmid according to any one of claims 1 to 5 and specifically expressing the gene in the seed, plant cell or plant.

9. A seed, plant cell or plant obtained by the method of claim 8.

10. A seed, plant cell or plant comprising the expression plasmid according to any one of claims 1 to 5.
